(19) Europäisches Patentamt
European Patent Office
Office européen des brevets

(11) Veröffentlichungsnummer: **0 311 878**
**A2**

(12) **EUROPÄISCHE PATENTANMELDUNG**

(21) Anmeldenummer: **88116445.3**

(51) Int. Cl.[4]: **C07D 219/08**

(22) Anmeldetag: **05.10.88**

(30) Priorität: **10.10.87 DE 3734383**

(43) Veröffentlichungstag der Anmeldung: **19.04.89 Patentblatt 89/16**

(84) Benannte Vertragsstaaten: **CH DE FR GB LI**

(71) Anmelder: **HOECHST AKTIENGESELLSCHAFT**
**Postfach 80 03 20**
**D-6230 Frankfurt am Main 80(DE)**

(72) Erfinder: **Dietz, Erwin, Dr.**
**St. Matthäus-Strasse 7**
**D-6233 Kelkheim Taunus(DE)**
Erfinder: **Hamal, Heinrich**
**Am Kalkofen 39**
**D-6237 Liederbach(DE)**
Erfinder: **Prokschy, Frank, Dr.**
**Drosselweg 3**
**D-6230 Frankfurt am Main 80(DE)**

(54) **Verfahren zur Herstellung von 2-(Arylamino)-3-carboxy-9(10H)-acridonen.**

(57) Verfahren zur Herstellung von 2-(Arylamino)-3-carboxy-9(10H)-acridonen der Formel A

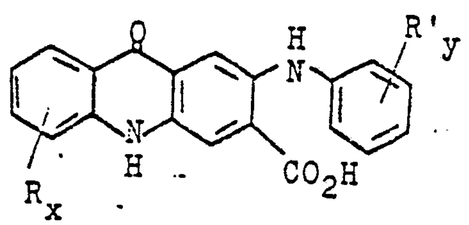

A

worin R und R′ Wasserstoff-, Fluor-, Chlor- oder Bromatome, Alkyl($C_1$-$C_4$)-, Oxalkyl($C_1$-$C_4$)- oder Carbonamidgruppen darstellen und x und y ganze Zahlen von 1 bis 4 bedeuten, indem man 2,5-Diarylaminoterephthalsäuren der Formel D

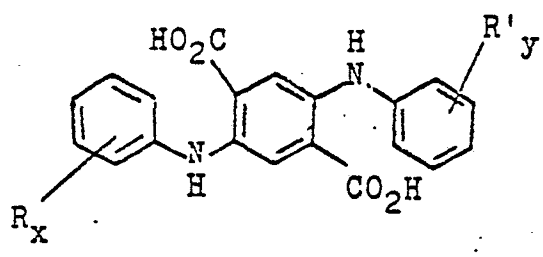

D

worin R, R′, x und y die vorstehend genannten Bedeutungen haben, in der 5- bis 15-fachen Gewichtsmenge Polyphosphorsäure ($P_4O_{10}$-Gehalt 80 bis etwa 85 %) oder eines sauren Polyphosphorsäureesters ($P_4O_{10}$-Gehalt 75 bis etwa 85 %) als wasserentziehendes Mittel bei etwa 60 °C bis etwa 100 °C cyclisiert.

EP 0 311 878 A2

Xerox Copy Centre

## Verfahren zur Herstellung von 2-(Arylamino)-3-carboxy-9(10H)-acridonen

Die vorliegende Erfindung betrifft ein verbessertes Verfahren zur Herstellung von 2-(Arylamino)-3-carboxy-9(10H)-acridonen (2-Arylamino-acridon-3-carbonsäuren) der allgemeinen Formel A

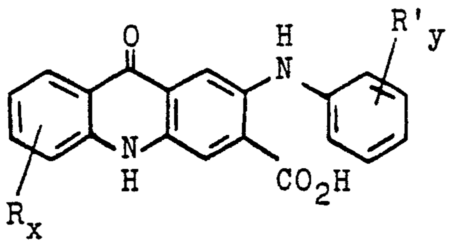

A

in welcher R und R′ Wasserstoff-, Fluor-, Chlor- oder Bromatome, Alkyl($C_1$-$C_4$)-, Oxalkyl($C_1$-$C_4$)- oder Carbonamidgruppen darstellen und x und y ganze Zahlen von 1 bis 4 bedeuten, in einem wasserentziehenden, sauren Medium bei mäßig hohen Temperaturen.

Verbindungen der genannten allgemeinen Formel A bzw. die entsprechenden Carbonsäurealkylester sind wichtige Vorstufen zur Herstellung von 2-(Arylamino)-9(10H)-acridonen der allgemeinen Formel B (US-PS 4 544 746, US-PS 4 258 190) und für Chinacridone der allgemeinen Formel C (DE-OS 2 165 647)

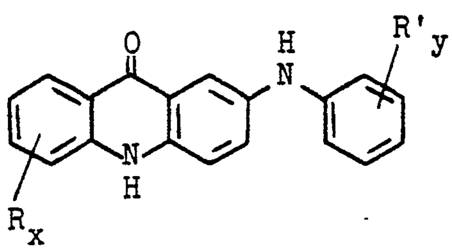

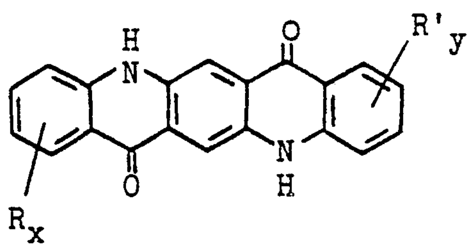

B C

in welchen R′ R′, x und y die vorstehend genannten Bedeutungen haben.

Verbindungen der genannten allgemeinen Formel B finden Verwendung als Mischkristallkomponenten zur Verbesserung der Licht- und Wetterechtheit von Chinacridonchinonpigmenten (US-PS 4 286 998); Chinacridone der genannten allgemeinen Formel C haben als Pigmente für die verschiedensten Anwendungsgebiete große Bedeutung.

In der DE-OS 2 165 647 bzw. GB-PS 1 382 259 wird bereits ein Verfahren zur Herstellung der bis damals unbekannten 2-Arylamino-acridon-3-carbonsäuren beschrieben, wobei 3,6-Dihydro-2,5-diarylamino-terephthalsäureester unter geeigneten Bedingungen zu den entsprechenden 1,4-Dihydro-2-arylamino-acridon-3-carbonsäureestern cyclisiert werden, diese dann zu den entsprechenden Acridonen dehydriert und diese wiederum zu Verbindungen der allgemeinen Formel A verseift werden. Der Nachteil dieses Verfahrens liegt in den geringen Ausbeuten an den Verbindungen A (50-60 %, bezogen auf das Dihydroterephthalsäurederivat).

In der US-PS 4 544 746 geht man bei der Synthese der Verbindungen der genannten allgemeinen Formel A von den 2,5-Diarylaminoterephthalsäuren der allgemeinen Formel D gemäß dem Reaktionsschema

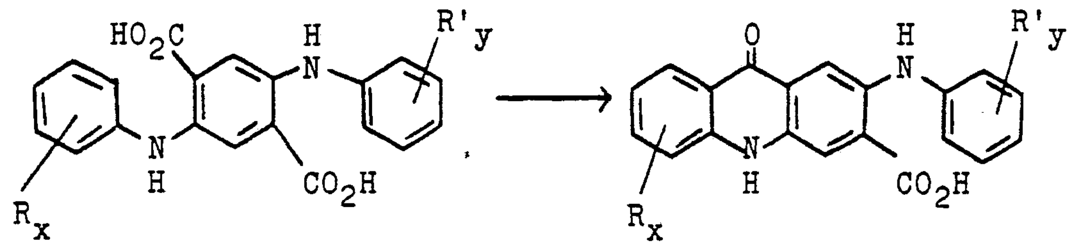

D A

(R, R′, x und y haben hierin die weiter oben angegebenen Bedeutungen) aus, die aus den entsprechenden 3,6-Dihydro-2,5-diarylamino-terephthalsäureestern durch Oxidation und anschließende Verseifung in hohen Ausbeuten erhältlich sind. Der Nachteil des letztgenannten Verfahrens liegt in der großen Menge Phosphorsäure (74,4-79,9 % $P_4O_{10}$), die als Ringschlußmedium für die 2,5-Diarylaminoterephthalsäuren verwendet wird (19-100 Gewichtsteile Phosphorsäure pro Teil 2,5-Diarylaminoterephthalsäure). In der zitierten US-PS 4 544 746 wird als Grund für diese Arbeitsweise angegeben, daß man unter Bedingungen, wie sie normalerweise zur Synthese von Chinacridonen der nachstehend genannten allgemeinen Formel C gewählt werden (US-PS 3 342 823), die Reaktion nicht auf der Zwischenstufe A anhalten kann (vgl. hierzu das nachstehende Reaktionsschema):

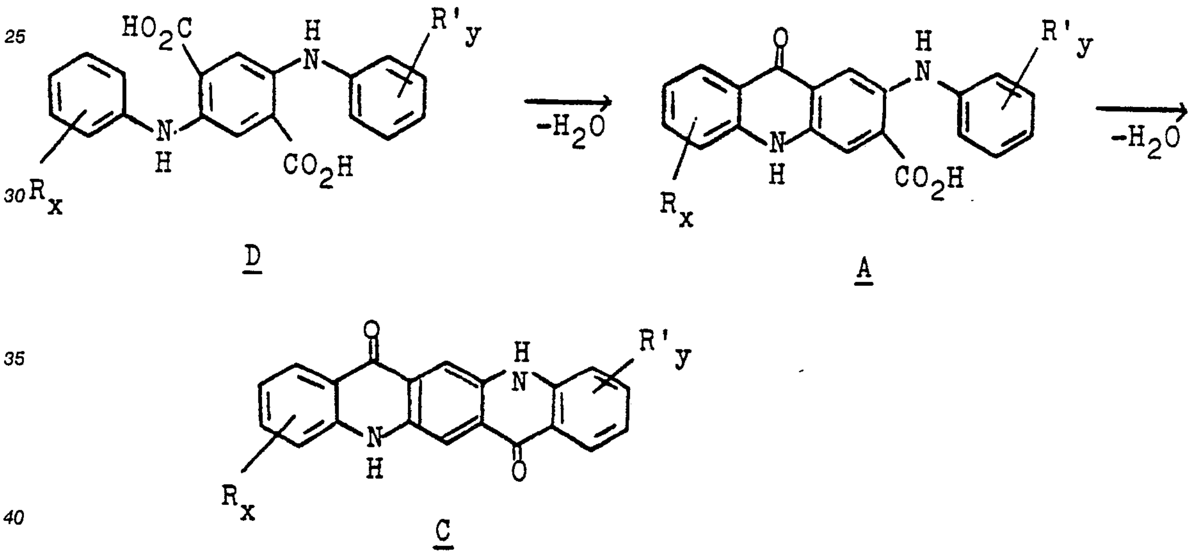


C

Im Hinblick auf die große Bedeutung der Verbindungen der genannten allgemeinen Formel (A) bzw. deren Carbonsäureester als Vorstufen für die Verbindungen der genannten allgemeinen Formeln B und C und aufgrund der erwähnten schwerwiegenden Nachteile der aus der DE-OS 2 165 647 (GB-PS 1 382 259) bzw. US-PS 4 544 746 bekannten Verfahren, bestand ein erhebliches Bedürfnis nach einem besseren Verfahren, welches die genannten Nachteile der bekannten Verfahren vermeidet.

Es würde nun überraschenderweise gefunden, daß man 2-(Arylamino)-3-carboxy-9(10H)-acridone der allgemeinen Formel A

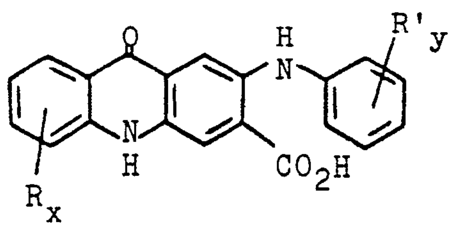

A

in welcher R und R′ Wasserstoff-, Fluor-, Chlor- oder Bromatome, Alkyl($C_1$-$C_4$)-, Oxalkyl($C_1$-$C_4$)- oder Carbonamidgruppen darstellen und x und y ganze Zahlen von 1 bis 4 bedeuten, in vorteilhafter Weise mit hohen Ausbeuten herstellen kann, indem man 2,5-Diarylaminoterephthalsäuren der allgemeinen Formel D

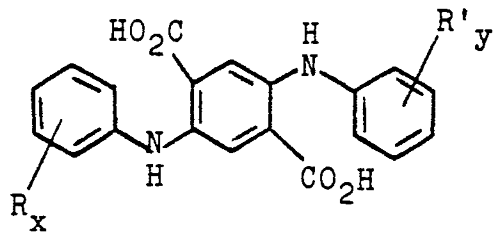

D

in welcher R, R′, x und y die genannten Bedeutungen haben, in der 5- bis 15-fachen, vorzugsweise 5- bis 8-fachen Gewichtsmenge Polyphosphorsäure ($P_4O_{10}$-Gehalt 80 bis etwa 85 %), oder eines sauren Polyphosphorsäureesters ($P_4O_{10}$-Gehalt 75 bis etwa 85 %, bevorzugt 78 bis 82 %) als wasserentziehendes Mittel bei Temperaturen von etwa 60° C bis etwa 100° C, vorzugsweise etwa 80° C bis etwa 90° C, cyclisiert.

Als saure Polyphosphorsäureester kommen hauptsächlich der Methyl-, Benzyl- und Phenylester in Frage.

Es zeigt sich, daß die Cyclisierung der Verbindungen der Formel D zu den Verbindungen der Formel A unter den angewandten Bedingungen zunächst relativ schnell verläuft.

Um die Aufarbeitung zu erleichtern, wird die Reaktion so lange fortgeführt bis keine Ausgangsverbindung der Formel D mehr vorhanden ist, was allerdings zur Folge hat, daß die erhaltenen Verbindungen der Formel A in geringen Mengen zu den Verbindungen der Formel C reagieren können.

Die Reaktionsführung erfolgt im allgemeinen so, daß die 2,5-Diarylaminoterephthalsäuren der genannten allgemeinen Formel D bei Reaktionstemperatur in das Ringschlußmedium eingetragen werden. Der Reaktionsverlauf wird dünnschichtchromatographisch verfolgt. Das Reaktionsende ist erreicht, wenn keine 2,5-Diarylaminoterephthalsäure im Ansatz mehr vorhanden ist. Die Reaktionszeit hängt im wesentlichen von der Temperatur und dem verwendeten Ringschlußmedium (wasserentziehenden Mittel) ab. Im Reaktionsprodukt sind auch gewisse Anteile an Chinacridonen der genannten allgemeinen Formel C nachzuweisen, deren Menge von der Reaktionstemperatur und der Art des Ringschlußmediums (wasserentziehendes Mittel) abhängig ist. Die Isolierung dieser ebenfalls wertvollen Verbindungen erfolgt in der Art, wie sie für das Abtrennen einer sauren von einer neutralen organischen Verbindung üblich ist und auch in der US-PS 4 544 746 beschrieben wird, nämlich durch Behandeln des Gemisches mit einer wäßrigen Alkalimetallhydroxidlösung. Dabei geht die saure Verbindung der Formel A in Lösung, während die Verbindung der Formel C durch Filtration abgetrennt wird. Anschließend wird das Filtrat angesäuert, wobei die Verbindung der Formel A ausfällt.

Das erfindungsgemäße Verfahren der Herstellung der Verbindungen der Formel A aus den Verbindungen der Formel D hebt sich von dem aus der US-PS 4 544 746 bekannten Verfahren dadurch vorteilhaft ab, daß zum einen bei niedrigeren Temperaturen gearbeitet wird, zum anderen ein wesentlich kleineres Verhältnis von Ringschlußmedium zu 2,5-Diarylaminoterephthalsäure (Formel D) angewandt wird, was ökologische Vorteile hat.

Die in den nachstehenden Beispielen angegebenen Mengen sind Gewichtsmengen; bei den Prozentangaben handelt es sich um Gewichtsprozente.

Beispiele

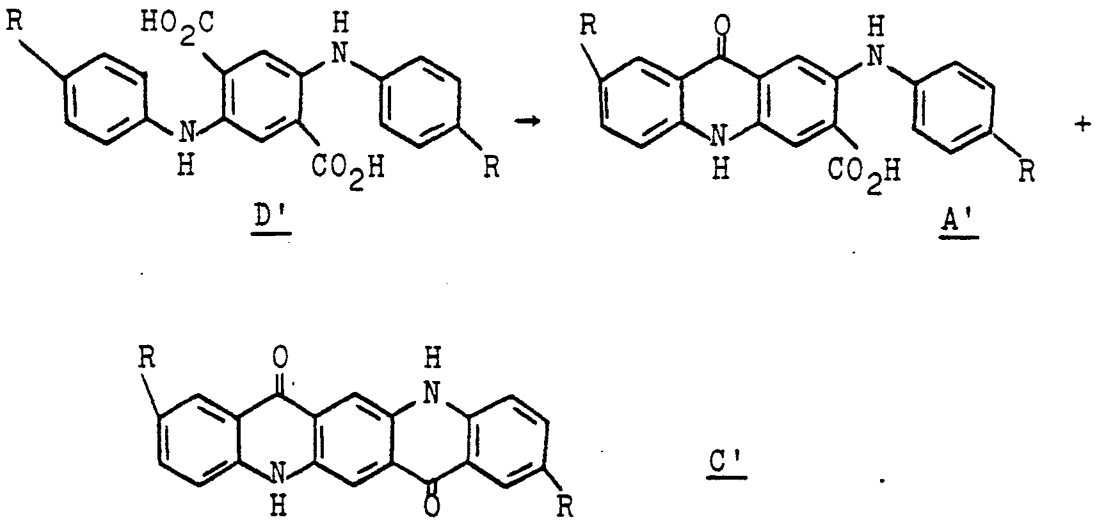

D' → A' + C'

a Teile Polyphosphorsäure (PPS) bzw. saurer Polyphosphorsäuremethylester (PPSMe) mit dem angegebenen Gehalt an $P_4O_{10}$ werden auf 70-75° C erwärmt; anschließend werden b Teile 2,5-Diarylaminoterephthalsäure in x Stunden so eingetragen, daß T (siehe nachstehende Tabelle) nicht überschritten wird; der Reaktionsansatz wird dann noch y Stunden bei Temperatur T nachgerührt.
Die Hydrolyse erfolgt durch Eingießen des Ansatzes in c Teile Wasser von 80° C. Anschließend wird das Hydrolysat noch 1-2 Stunden bei 80-90° C verrührt. Nach Absaugen,

Waschen und Trocknen erhält man d Teile eines Gemisches, welches überwiegend aus 2-Arylaminoacridon-3-carbonsäure der Formel A' mit einem geringen Anteil an Chinacridon der Formel C' besteht.

Die vorstehend beschriebene Umsetzung liegt den nachstehenden Tabellenbeispielen 1 bis 14 zugrunde.

| Bsp. | R | a | PPS bzw. PPSMe | % $P_4O_{10}$ | T | b | x | y | c | d | % A' | % C' |
|---|---|---|---|---|---|---|---|---|---|---|---|---|
| 1 | H | 1200 | PPS | 80,7 | 85° C | 200 | 1,5 | 1 | 3600 | 180 | 91,6 | 8,4 |
| 2 | H | 800 | PPS | 85,0 | 85° C | 100 | 0,5 | 3 | 4000 | 89 | 75 | 25 |
| 3 | H | 1200 | PPS | 81,4 | 85° C | 200 | 3 | 1 | 3600 | 181 | 84 | 16 |
| 4 | H | 1030 | PPS | 80,0 | 80° C | 172 | 2 | 4 | 3100 | 160 | 93,3 | 6,7 |
| 5 | H | 1200 | PPS | 80,8 | 85° C | 200 | 2 | 1 | 3600 | 180 | 96,3 | 3,7 |
| 6 | H | 1200 | PPS | 81,7 | 85° C | 200 | 2,75 | 1 | 3600 | 180 | 87 | 13 |
| 7 | H | 600 | PPSMe | 78,5 | 85° C | 100 | 2 | 1 | 1800 | 92 | 76 | 24 |
| 8 | H | 150 | PPSMe | 81,8 | 85° C | 25 | 0,5 | 1 | 500 | 22 | 79 | 21 |
| 9 | H | 250 | PPSMe | 78,1 | 83° C | 42 | 0,25 | 4 | 750 | 38 | 84 | 16 |
| 10 | $CH_3$ | 1200 | PPS | 80,7 | 80° C | 200 | 1 | 3,25 | 3600 | 180 | 94 | 6 |
| 11 | $CH_3$ | 1200 | PPS | 82,3 | 80° C | 200 | 1,5 | 3 | 3600 | 186 | 91 | 9 |
| 12 | $CH_3$ | 150 | PPSMe | 78,9 | 85° C | 25 | 0,25 | 4 | 500 | 23 | 84 | 16 |
| 13 | Cl | 200 | PPSMe | 78,5 | 87° C | 20 | 0,5 | 7 | 600 | 17 | 45 | 55 |
| 14 | Cl | 500 | PPS | 80,7 | 90° C | 100 | 1,25 | 5 | 1500 | 38 | 77 | 23 |

Verwendet man anstelle der in der vorstehenden Tabelle angegebenen Mengen an saurem Polyphosphorsäuremethylester solche Mengen an saurem Polyphosphorsäurebenzyl- oder -phenylester, daß der Prozentgehalt dieser Ester an $P_4O_{10}$ gleich ist, so erhält man die Verbindungen der Formel A' in befriedigenden Ausbeuten.

Das Abtrennen des Chinacridons aus dem Gemisch erfolgt derart, daß man den feuchten Filterkuchen, den man nach dem Absaugen und Neutralwaschen des Hydrolysats erhält, in der 35-fachen Gewichtsmenge (bezogen auf Trockengehalt) Wasser anrührt, mit 30%iger Natronlauge einen pH-Wert von 12 einstellt und 2 Stunden bei einer Temperatur von 60° C rührt. Anschließend wird abgesaugt und der Rückstand gewaschen, wobei man das Chinacridon der Formel C' erhält. Aus der Mutter- und Waschlauge erhält man nach Einstellen eines pH-Wertes von 4 mittels Essigsäure, Absaugen, Waschen und Trocknen die

entsprechende 2-Arylamino-acridon-3-carbonsäure der Formel A′.

Ähnliche Ergebnisse erzielt man, wenn man als Ausgangsverbindungen der Formel D′ solche einsetzt, bei welchen R eine Ethylgruppe oder ein Fluor- oder Bromatom bedeutet.

## Ansprüche

1. Verfahren zur Herstellung von 2-(Arylamino)-3-carboxy-9(10H)-acridonen der allgemeinen Formel A

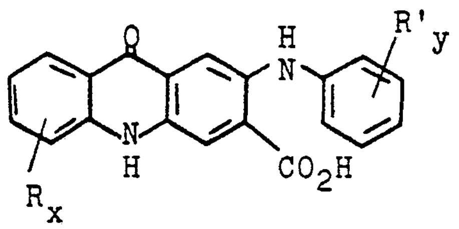

A

in welcher R und R′ Wasserstoff-, Fluor-, Chlor- oder Bromatome, Alkyl($C_1$-$C_4$)-, Oxalkyl($C_1$-$C_4$)- oder Carbonamidgruppen darstellen und x und y ganze Zahlen von 1 bis 4 bedeuten, dadurch gekennzeichnet, daß man 2,5-Diarylaminoterephthalsäuren der allgemeinen Formel D

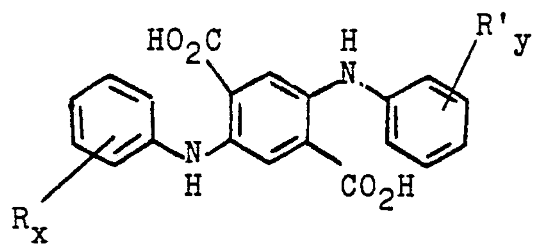

D

in welcher R, R′, x und y die vorstehend genannten Bedeutungen haben, in der 5- bis 15-fachen Gewichtsmenge Polyphosphorsäure ($P_4O_{10}$-Gehalt 80 bis etwa 85 %) oder eines sauren Polyphosphorsäureesters ($P_4O_{10}$-Gehalt 75 bis etwa 85 %) als wasserentziehendes Mittel bei Temperaturen von etwa 60 °C bis etwa 100 °C cyclisiert.

2. Verfahren nach Anspruch 1, dadurch gekennzeichnet, daß man die Cyclisierung bei Temperaturen von etwa 80 °C bis etwa 90 °C vornimmt.

3. Verfahren nach mindestens einem der Ansprüche 1 und 2, dadurch gekennzeichnet, daß man die Cyclisierung in der 5- bis 8-fachen Gewichtsmenge Polyphosphorsäure oder eines sauren Polyphosphorsäureesters vornimmt.

4. Verfahren nach mindestens einem der Ansprüche 1 bis 3, dadurch gekennzeichnet, daß man die Cyclisierung in Gegenwart eines sauren Polyphosphorsäureesters mit einem $P_4O_{10}$-Gehalt von 78 bis 82 % vornimmt.

5. Verfahren nach mindestens einem der Ansprüche 1 bis 4, dadurch gekennzeichnet, daß man eine Verbindung der allgemeinen Formel D cyclisiert, in welcher R und/oder R′ die Methyl- oder Ethylgruppe oder ein Fluor-, Chlor- oder Bromatom bedeutet.